# EUROPEAN PATENT APPLICATION

(11) **EP 4 717 216 A1**
(43) Date of publication of application: **01.04.2026**
(21) Application number: 24855753.0
(22) Date of filing: 18.08.2024
(51) Int. Cl.: A61B 18/18, A61N 5/06

(54) **SKIN CARE DEVICE**

(30) Priority: 18.08.2023 CN 202322243927 U
(71) Applicant: Hangzhou Ulike Technology Co., Ltd., Hangzhou, Zhejiang 310051 (CN)
(72) Inventor: HE, Dengshi, Hangzhou, Zhejiang 310051 (CN)
(74) Representative: Winter, Brandl - Partnerschaft mbB
(86) International application number: PCT/CN2024/112937
(87) International publication number: WO 2025/040030

(57) **Abstract**

A skin care device, comprising a housing (100), a light source assembly (200), a circuit board (300), and a heat dissipation assembly (400). The housing (100) is provided with a light outlet (11). The light source assembly (200) is arranged in the housing (100), and comprises a reflective component (21) and at least two light sources (22); the light outlet (11) is located on the side of the light sources (22) in a first direction (H1); and the reflective component (21) is at least partially located on the side of the light sources (22) facing away from the light outlet (11). At least one side of the light sources (22) in a second direction (H2) is provided with the circuit board (300), the second direction (H2) and the first direction (H1) are mutually orthogonal, and different loops are between different light sources (22) and the circuit board (300). The heat dissipation assembly (400) is at least partially located on the side of the reflective component (21) facing away from the light outlet (11).

## Description

This application claims the priority of Chinese Patent Application No. 202322243927.5, entitled "skin care device", filed with the China Patent Office on August 18, 2023, the entire content of which is incorporated herein by reference.

### TECHNICAL FIELD

The present application relates to the field of beauty devices, and in particular, to a skin care device.

### BACKGROUND

Hair removal devices and photon rejuvenation devices are commonly used skin care devices in daily life. These skin care devices achieve effects such as hair removal and photon skin rejuvenation on skin of a user, mainly by irradiating it with intense pulsed light (IPL).

### SUMMARY

### Technical problem

In the related technologies, taking a hair removal device as an example, a skin care device generally includes only one lamp tube as an IPL light source, resulting in poor skin care effects of the skin care device.

### Technical solution

An embodiment of the present application provides a skin care device, including:
a housing, where a light outlet is formed on the housing;
a light source assembly arranged inside the housing, where the light source assembly includes a light reflector and at least two light sources, the light outlet is located on a side of the light sources along a first direction, the light reflector is at least partially located on a side of the light sources facing away from the light outlet to reflect part of light emitted from the light sources towards the light outlet;
a circuit board arranged inside the housing, where the circuit board is arranged on at least one side of the light sources along a second direction, the second direction is perpendicular to the first direction, and the circuit board is electrically connected to the light sources; and
a heat dissipation assembly arranged inside the housing, where the heat dissipation assembly is at least partially located on a side of the light reflector facing away from the light outlet.

### Beneficial Effects

In the embodiments of the present application, the light source assembly improves the care effect of the skin care device by emitting light through multiple light sources. For example, the multiple light sources are switched on or off synchronously to allow the light source assembly to perform skin care at a high maximum power, thereby enhancing the care effect of the skin care device. Alternatively, different light sources emit light either independently or in combination, such that the light source assembly provides at least three light output modes and a suitable light output mode is selected according to the actual needs of the user, thereby further improving the care effect of the skin care device. In addition, since the light sources are main heat source of the skin care device, heat generated at the light source assembly increases significantly with the increase in the number of light sources. Compared with a configuration in which a circuit board is placed between a side of the light reflector facing away from the light outlet, and the heat dissipation assembly, in the embodiments of the present application, the circuit board is located on another side of the light sources, so as to allow the heat dissipation assembly to be positioned closer to the light source assembly, which shortens a heat dissipation path, thereby providing an effective heat dissipation for the light source assembly with multiple light sources, avoiding abnormal operation of the light source assembly due to overheating of the multiple light sources during operation, and ultimately improving the skin care effect of the skin care device.

### BRIEF DESCRIPTION OF THE DRAWINGS

FIG. 1 is a perspective view of a skin care device according to an embodiment of the present application.
FIG. 2 is a first schematic structural view of the skin care device shown in FIG. 1.
FIG. 3 is a second schematic structural view of a circuit board of the skin care device shown in FIG. 1.
FIG. 4 is a second schematic structural view of a conductive bracket of the skin care device shown in FIG. 1.
FIG. 5 is a first schematic positional view of light sources of the skin care device shown in FIG. 1.
FIG. 6 is a second schematic positional view of light sources of the skin care device shown in FIG. 1.
FIG. 7 is a second schematic structural view of a light reflector of the skin care device shown in FIG. 1.
FIG. 8 is a schematic structural view of light sources of the skin care device shown in FIG. 2.
FIG. 9 is a schematic structural view of light sources shown in FIG. 8 after installation of spacers.
FIG. 10 is a third schematic structural view of the conductive bracket of the skin care device shown in FIG. 1.
FIG. 11 is a schematic structural view of the conductive bracket of the skin care device shown in FIG. 2.
FIG. 12 is a schematic structural view of the conductive bracket shown in FIG. 11.
FIG. 13 is a fourth schematic structural view of the conductive bracket of the skin care device shown in FIG. 1.
FIG. 14 is a fifth schematic structural view of the conductive bracket of the skin care device shown in FIG. 1.
FIG. 15 is a sixth schematic structural view of the conductive bracket of the skin care device shown in FIG. 1.
FIG. 16 is a top view of the skin care device shown in FIG. 1.
FIG. 17 is a cross-sectional view of the skin care device shown in FIG. 16 taken along line A-A.
FIG. 18 is an enlarged view of part X in FIG. 17.
FIG. 19 is a cross-sectional view of the skin care device shown in FIG. 16 taken along line B-B.

### DETAILED DESCRIPTION

Hair removal devices and photon rejuvenation devices are commonly used skin care devices in daily life. These skin care devices achieve effects such as hair removal and photon skin rejuvenation on skin of a user, mainly by irradiating it with intense pulsed light (IPL).

In the related technologies, taking a hair removal device as an example, a skin care device generally includes only one lamp tube as an IPL light source, resulting in poor skin care effects of the skin care device.

In view of the above, in the embodiments of the present application, a skin care device is provided to improve the care effect on the skin. The skin care device is a hair removal device or a rejuvenation device for performing hair removal or skin rejuvenation, respectively. Alternatively, the skin care device is another type of beauty device having hair removal or skin rejuvenation functions, and the embodiments of the present application are not limited thereto.

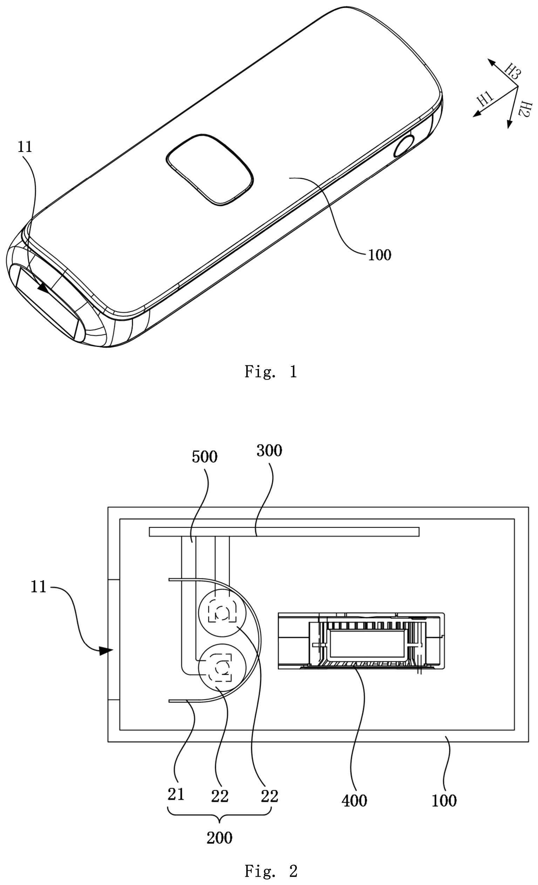

Referring to FIG. 1 and FIG. 2. FIG. 1 is a perspective view of a skin care device according to an embodiment of the present application, and FIG. 2 is a first schematic structural view of the skin care device shown in FIG. 1. The skin care device includes a housing 100, a light source assembly 200, a circuit board 300, and a heat dissipation assembly 400.

A light outlet 11 is formed on the housing 100. The light source assembly 200 is arranged inside the housing 100. The light source assembly 200 includes a light reflector 21 and at least two light sources 22. The light outlet 11 is located on a side of the light sources 22 along a first direction H1. The light reflector 21 is at least partially located on a side of the light sources 22 facing away from the light outlet 11 to reflect part of light emitted from the light sources 22 towards the light outlet 11. The circuit board 300 is arranged inside the housing 100. The circuit board 300 is arranged on at least one side of the light sources 22 along a second direction H2. The second direction H2 is perpendicular to the first direction H1. The circuit board 300 is electrically connected to the light sources 22. The heat dissipation assembly 400 is arranged inside the housing 100. The heat dissipation assembly 400 is at least partially located on a side of the light reflector 21 facing away from the light outlet 11.

It can be understood that, in the embodiment of the present application, the care effect of the skin care device is improved by emitting light through multiple light sources 22. For example, the multiple light sources 22 are switched on or off synchronously to allow the light source assembly 200 to perform skin care at a high maximum power, thereby enhancing the care effect of the skin care device. Alternatively, different light sources 22 emit light either independently or in combination, so that the light source assembly 200 provides at least three light output modes and a suitable light output mode is selected according to the actual needs of the user, thereby further improving the care effect of the skin care device.

In addition, since the light sources 22 are main heat source of the skin care device, heat generated at the light source assembly 200 increases significantly with the increase in the number of light sources 22. Compared with a configuration in which a circuit board 300 is placed between a side of the light reflector 21 facing away from the light outlet 11, and the heat dissipation assembly 400, in the embodiment of the present application, a circuit board 300 is arranged on another side of the light sources 22 to allow the heat dissipation assembly 400 positioned closer to the light source assembly 200, which shortens a heat dissipation path, thereby providing an effective heat dissipation for the light source assembly 200 with multiple light sources 22, avoiding abnormal operation of the light source assembly 200 due to overheating of the multiple light sources 22 during operation, and ultimately improving the skin care effect of the skin care device.

The shape of the skin care device is various, for example, it is in a shape of a hair dryer, strip, flat small rectangle, circle, or ellipse. For the skin care device in a shape of a substantial strip, the first direction H1 corresponds to a length direction of the skin care device, and the second direction H2 corresponds to a thickness direction of the skin care device. Alternatively, the first direction H1 corresponds to the length direction of the skin care device, and the second direction H2 corresponds to a width direction of the skin care device. Alternatively, the first direction H1 corresponds to the width direction of the skin care device, and the second direction H2 corresponds to the thickness direction of the skin care device. The embodiments of the present application are not limited thereto.

In the following description, the embodiments of the present application are mainly illustrated by taking the skin care device in a shape of a substantial strip as an example. That is, the embodiments of the present application will continue to use an example in which the first direction H1 corresponds to the length direction of the skin care device and the second direction H2 corresponds to the thickness direction of the skin care device to explain and describe the technical solutions of the embodiments of the present application.

The following provides illustrative examples of several optional installation structures of the light source assembly 200 to further explain the technical solutions of the embodiments of the present application.

In some embodiments, the skin care device further includes a conductive bracket 500. The conductive bracket 500 is mounted on the circuit board 300 and connected to the light sources 22, so that the light sources 22 are supported on and electrically connected to the circuit board 300. Moreover, the conductive bracket 500 realizes both a mechanical connection and fixation between the light sources 22 and the circuit board 300, and an electrical connection between the light sources 22 and the circuit board 300.

The conductive bracket 500 is made of an electrically conductive metal material. For example, the conductive bracket 500 is made of copper, aluminum, silver, gold, or nickel, or the like. The conductive bracket 500 is also made of another non-metallic conductive material, such as graphite or conductive rubber. The embodiments of the present application are not limited thereto.

In some embodiments, multiple conductive brackets 500 are provided. All end portions of the conductive brackets 500 connected to the circuit board 300 are located on the same side of the light source assembly 200 along the second direction H2.

For example, only one circuit board 300 is provided. The one circuit board 300 is positioned on a first side of the light source assembly 200 along the second direction H2. In this case, one circuit board 300 is located on either an upper side or a lower side of the light source assembly 200 in the thickness direction of the skin care device. Thus, lower ends of all the conductive brackets 500 are connected to the light sources, and all upper ends thereof are connected to the circuit board 300 located on the upper side of the light source assembly 200. Alternatively, the upper ends of all the conductive brackets 500 are connected to the light sources, and the lower ends thereof are connected to the circuit board 300 located on the lower side of the light source assembly 200. In this way, in the skin care device, the number of circuit board 300 required for installing the light sources 22 is reduced to make the installation of the circuit board 300 inside the skin care device convenient.

For another example, multiple circuit boards 300 are provided. Different circuit boards 300 are located on different sides of the light source assembly 200 along the second direction H2. In this case, all the conductive brackets 500 are connected to the circuit board located on a first side, or in other words, on the same side of the light source assembly 200 along the second direction H2. The embodiments of the present application are not limited thereto.

Alternatively, multiple conductive brackets 500 are provided. The end portions of the conductive brackets 500 connected to the circuit board 300 are located on different sides of the light source assembly 200 along the second direction H2.

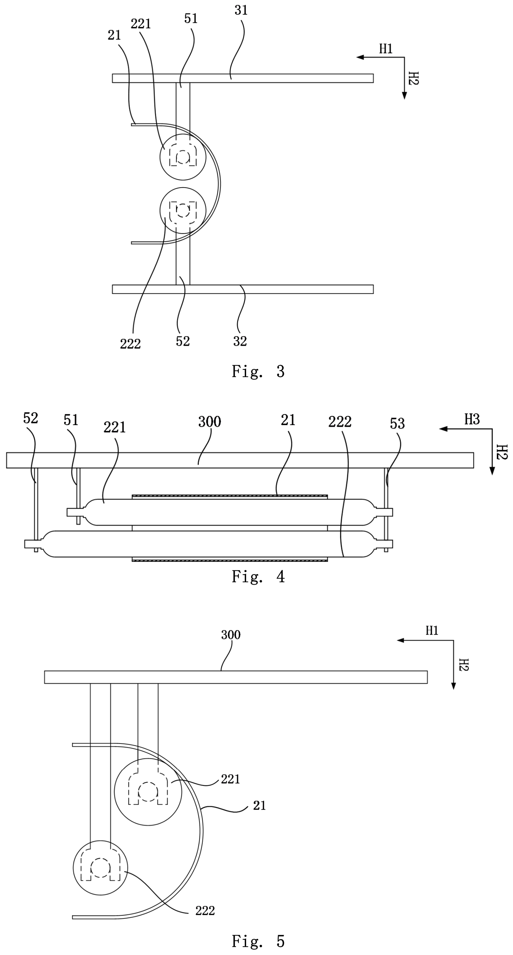

For example, please continue to refer to FIG. 3, which is a second schematic structural view of a circuit board of the skin care device shown in FIG. 1. The circuit board 300 includes a first sub-circuit board 31 and a second sub-circuit board 32. The first sub-circuit board 31 and the second sub-circuit board 32 are respectively located on different sides of the light source assembly 200 along the second direction H2. For example, the first sub-circuit board 31 is located on the upper side of the light source assembly 200 in the thickness direction, and the second sub-circuit board 32 is located on a lower side of the light source assembly 200 in the thickness direction.

In actual assembly, different conductive brackets 500 are distributed and mounted on different circuit boards 300 to avoid problems such as short circuits or leakage caused by overly dense conductive brackets 500, and also facilitate the installation of the conductive brackets 500 of different light sources 22 onto adjacent circuit boards 300.

For another example, the circuit board 300 is a flexible printed circuit board (FPC). The flexible printed circuit board 300 at least partially surrounds the light source assembly 200. For example, the flexible circuit board 300 surrounds an upper side in the thickness direction, a left side in the width direction, and a lower side in the thickness direction of the light source assembly 200, such that some conductive brackets 500 are connected to an upper portion of the flexible circuit board 300 and others are connected to a lower portion of the conductive brackets 500.

Hereinafter, the conductive bracket 500 according to the embodiments of the present application is further illustrated in connection with the light sources 22.

The at least two light sources 22 include a first light source 221 and a second light source 222. Therefore, at least three different light output modes are achieved through the first light source 221 and the second light source 222. Accordingly, the number of light sources 22 is three, four, five, or six, and the first light source 221, and the second light source 222 are any two of the multiple light sources 22. The embodiments of the present application are not limited thereto.

Please continue to refer to FIG. 4, which is a second schematic structural view of the conductive bracket of the skin care device shown in FIG. 1. The conductive bracket 500 includes a first conductive bracket 51, a second conductive bracket 52, and a third conductive bracket 53. Positive electrodes of both the first light source 221 and the second light source 222 are electrically connected to the third conductive bracket 53. A negative electrode of the first light source 221 is electrically connected to the first conductive bracket 51, and a negative electrode of the second light source 222 is electrically connected to the second conductive bracket 52.

Accordingly, the third conductive bracket 53 is understood as a common positive electrode, which reduces the number of the conductive brackets 500 required between the light sources 22 and the circuit board 300, thereby simplifying the structure of the skin care device, and reducing assembly difficulty and manufacturing costs of the skin care device.

It can also be understood that the circuit board 300, the third conductive bracket 53, the first light source 221, and the first conductive bracket 51 together form a first loop; while the circuit board 300, the third conductive bracket 53, the second light source 222, and the second conductive bracket 52 together form a second loop. Therefore, independent control of the first light source 221 and the second light source 222 is achieved by arranging a switch at a corresponding position on the first conductive bracket 51, and arranging another switch at a corresponding position on the second conductive bracket 52.

Alternatively, the conductive bracket 500 includes a first conductive bracket 51, a second conductive bracket 52, and a third conductive bracket 53. Negative electrodes of both the first light source 221 and the second light source 222 are electrically connected to the third conductive bracket 53. A positive electrode of the first light source 221 is electrically connected to the first conductive bracket 51, and a positive electrode of the second light source 222 is electrically connected to the second conductive bracket 52.

Accordingly, the third conductive bracket 53 is understood as a common negative electrode, which reduces the number of the conductive brackets 500 required between the light sources 22 and the circuit board 300, thereby simplifying the structure of the skin care device, and reducing assembly difficulty and manufacturing costs of the skin care device.

It can also be understood that the circuit board 300, the third conductive bracket 53, the first light source 221, and the first conductive bracket 51 together form a first loop; while the circuit board 300, the third conductive bracket 53, the second light source 222, and the second conductive bracket 52 together form a second loop. Therefore, a switch is arranged at a corresponding position on the first conductive bracket 51, and another switch is arranged at a corresponding position on the second conductive bracket 52 to allow independent control of the first light source 221 and the second light source 222.

Accordingly, in some other embodiments, multiple conductive brackets 500 are provided. Different light sources 22 are mounted on the circuit board 300 via different conductive brackets 500. In this way, when any one conductive bracket 500 is detached or damaged, the remaining conductive brackets 500 still supply power to the corresponding light sources 22 to ensure the normal operation of other light sources 22 of the skin care device. Moreover, such an arrangement increases the light output power (e.g., power for hair removal) and/or the diversity of light output functions (e.g., the diversity of hair removal functions). In other words, at least two light sources 22 are provided, and these light sources 22 are mounted and fixed to the circuit board 300 via different conductive brackets 500. When a high hair removal power is required, multiple light sources 22 are switched on simultaneously; when a low hair removal power is required, a single light source 22 is switched on. Alternatively, at least two lamp tubes of at least two light sources 22 alternately emit light to increase the total light output power while reducing or avoiding the increase in the loss of the single light source 22.

It can further be understood that, in some other embodiments, the positive electrodes of all light sources 22 are connected to a same conductive bracket 500, and the negative electrodes of all the light sources 22 are connected to another conductive bracket 500. Thus, all the light sources 22 are mounted through the same conductive brackets 500 to allow the light source assembly 200 to operate at a high maximum power. Meanwhile, since all the light sources 22 are electrically connected through the same conductive brackets 500, the current transmission among all the light sources 22 is uniform, resulting in a consistent brightness of all the light sources 22 and a uniform brightness at various positions of the light outlet 11.

Taking again the case where multiple conductive brackets 500 are provided as an example. A distance between two adjacent conductive brackets 500 is greater than or equal to 0.8 mm and less than or equal to 10 mm, so as to prevent issues such as electric leakage caused by a small distance between two adjacent conductive brackets 500 and prevent a waste of the space within the skin care device due to a large distance between two adjacent conductive brackets 500.

For instance, the distance between two adjacent conductive brackets 500 configured to support different light sources 22 is 0.8 mm, 0.97 mm, 1.44 mm, 1.98 mm, 2.12 mm, 2.76 mm, 3.11 mm, 3.94 mm, 4.37 mm, 5.44 mm, 6.98 mm, 7.22 mm, 8.73 mm, 9.14 mm, or 10 mm. The embodiments of the present application are not limited thereto.

Hereinafter, the conductive bracket 500 will be further described in conjunction with at least two light sources 22 and the light reflector 21.

The multiple light sources 22 are parallel to one another. For example, each light source 22 comprises a lamp tube, such as a xenon lamp tube, and each lamp tube is arranged to extend along a third direction H3, that is, a length direction of each lamp tube is parallel to the third direction H3. The first direction H1, the second direction H2, and the third direction H3 are mutually orthogonal.

It can be understood that, compared with a scattered and disordered arrangement of multiple light sources 22, in the embodiment of the present application, arranging the multiple light sources 22 in parallel allows an overall light emission from the multiple light sources 22 more uniform. Taking again the case where the skin care device is a hair removal device or a skin rejuvenation device as an example, the brightness at various positions of the light outlet 11 is uniform, to prevent some areas of the skin from subjected to insufficient hair removal or rejuvenation due to low brightness, thereby improving the effect of hair removal or skin rejuvenation

Continuing with the example in which the first direction H1 corresponds to the length direction of the skin care device, the second direction H2 corresponds to the thickness direction of the skin care device, and the third direction H3 corresponds to the width direction of the skin care device. Accordingly, depending on the shape of the housing 100 of the skin care device, specific orientations of the first direction H1, the second direction H2, and the third direction H3 are varied, and the embodiments of the present application are not limited thereto.

In some embodiments, distances between the multiple light sources 22 and the light outlet 11 are equal. It can be understood that the intensity of light emitted from light sources 22 decreases with the increase in the radiation distance. In the embodiments of the present application, since the distances between the multiple light sources 22 and the light outlet 11 are equal, the brightness at various positions of the light outlet 11 is uniform. Taking again the case where the skin care device is a hair removal device or a skin rejuvenation device as an example, this arrangement prevents some areas of the skin from subjected to insufficient hair removal or rejuvenation due to low brightness, thereby improving the effect of hair removal or skin rejuvenation.

It can also be understood that the multiple light sources 22 are parallel to one another, while the distances between the multiple light sources 22 and the light outlet 11 are inequal. Alternatively, the multiple light sources 22 are not parallel to one another, while the distances between the multiple light sources 22 and the light outlet 11 are equal. Alternatively, the multiple light sources 22 are parallel to one another, and the distances between the multiple light sources 22 and the light outlet 11 are equal. The embodiments of the present application are not limited thereto.

For example, please continue to refer to FIG. 5, which is a first schematic positional view of light sources of the skin care device shown in FIG. 1. Continuing with the example where the at least two light sources 22 include a first light source 221 and a second light source 222. A distance between the first light source 221 and the light outlet 11 is greater than a distance between the second light source 222 and the light outlet 11. In other words, the first light source 221 is closer to the light outlet 11 than the second light source 222. In this case, a power of the first light source 221 is greater than a power of the second light source 222, so that the difference between an intensity of light emitted by the first light source 221 to the light outlet 11 and an intensity of light emitted by the second light source 222 to the light outlet 11 is reduced or even nearly avoided, resulting in a uniform brightness at various positions of the light outlet 11.

Next, the positional arrangement of the multiple light sources 22 will be further described in conjunction with the light reflector 21.

A reflective cavity is provided at the light reflector 21. An opening of the reflective cavity faces the light outlet 11. At least two light sources 22 are mounted inside the reflective cavity. In this way, the light emitted by the light sources 22 in 360° directions is reflected and concentrated toward the light outlet 11 through the reflective cavity to improve the light output efficiency of the light source assembly 200 or increase the brightness at the light outlet 11.

The at least two light sources 22 are arranged at an interval along the second direction H2.

For example, continuing with the previous example where the distances between the light sources 22 and the light outlet 11 are equal and/or the light sources 22 are parallel to each other. The two light sources 22 are arranged in a staggered manner along the thickness direction of the skin care device and arranged to extend along the width direction of the skin care device, resulting in a uniform brightness at various positions of the light outlet 11.

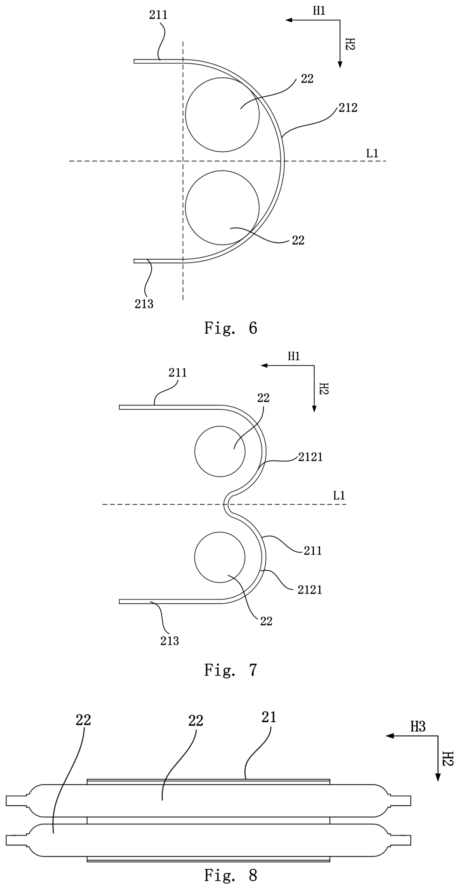

Please continue to refer to FIG. 6, which is a second schematic positional view of light sources of the skin care device shown in FIG. 1. In some embodiments, along the second direction H2, the reflective cavity is symmetrical about a first axis L1, where the first axis L1 is parallel to the first direction H1. All the light sources 22 are arranged symmetrically about the first axis L1. Because the reflective cavity is symmetrical about the first axis L1, when the light sources 22 are symmetrically arranged about the first axis L1, the light reflected by an inner wall of the reflective cavity from the multiple light sources 22 is uniform.

In some embodiments, the light reflector 21 includes a first straight portion 211, a connection portion 212, and a second straight portion 213 sequentially connected. The first straight portion 211 and the second straight portion 213 are arranged at an interval along the second direction H2, and the connection portion 212 faces the light outlet 11, so that the first straight portion 211, the connection portion 212, and the second straight portion 213 are enclosed to form a reflective cavity with an opening facing the light outlet 11. Part of the light emitted by the light sources 22 passes between the first straight portion 211 and the second straight portion 213 towards the light outlet 11, while the other part of the light emitted by the light sources 22 is reflected by one or more of the connection portion 212, the first straight portion 211, and the second straight portion 213 towards the light outlet 11.

In some embodiments, the connection portion 212 includes a curved surface. One end of the curved surface in a circumferential direction is connected to the first straight portion 211, and the other end of the curved surface in the circumferential direction is connected to the second straight portion 213. Alternatively, it can be understood that the first straight portion 211, the second straight portion 213, and the connection portion 212 form a U-shaped structure, with the connection portion 212 located at the middle of the U-shape structure. Since the connection portion 212 is in a shape of a parabola, the light emitted by the multiple light sources 22 is effectively concentrated, thereby increasing the brightness at the light outlet 11 and improving the uniformity of brightness at various positions of the light outlet 11.

Optionally, please continue to refer to FIG. 7, which is a second schematic structural view of a light reflector of the skin care device shown in FIG. 1. The connection portion 212 further includes at least two light-concentrating curved surfaces 2121. Each light-concentrating curved surface 2121 corresponds to one light source 22 and provides an effective light-concentrating effect for that light source 22. The embodiments of the present application are not limited thereto.

In some embodiments, the connection portion 212 are arranged to surround the at least two light sources 22. One end of the connection portion 212 connected to the first straight portion 211 and one end of the connection portion 212 connected to the second straight portion 213 are both protruded from a side of the at least two light sources 22 close to the light outlet 11.

Alternatively, it can be understood that, along the first direction H1, the multiple light sources 22 are located on an inner side of the connection portion 212, to allow the connection portion 212 to provide an effective light-concentrating effect for the multiple light sources 22 and improve the uniformity of brightness at various positions of the light outlet 11.

It can be understood that the light sources 22 are completely located within a space enclosed by the connection portion 212. Alternatively, the light sources 22 are partially located within the space enclosed by the connection portion 212. The embodiments of the present application are not limited thereto.

For example, please continue to refer to FIG. 8, which is a schematic structural view of light sources of the skin care device shown in FIG. 2. Continuing with the example where the light sources 22 are lamp tubes, a length direction of the lamp tubes, the first direction H1, and the second direction H2 are mutually orthogonal. At least one end of each lamp tube along its length direction is located outside the connection portion 212. Along the length direction of the lamp tubes, a length of a portion at each end of each of the lamp tubes located outside the connection portion 212 is less than or equal to one-fifth of a length of each of the lamp tubes.

For example, along the length direction of the lamp tubes, the length of a portion at each end of each of the lamp tubes located outside the connection portion 212 is one-fifth, one-sixth, one-seventh, or one-eighth of the length of each of the lamp tubes. The embodiments of the present application are not limited thereto.

It can be understood that, when the lamp tubes are in operation, the brightness at both ends along the length direction of the lamp tubes is low. Therefore, locating the portions of the lamp tubes with a low brightness outside the light reflector 21 not only prevents a waste of the light emitted by the lamp tubes, but also allows pins of the lamp tubes to be located outside the connection portion 212 to facilitate the connection with the conductive bracket 500, thereby reducing the difficulty of assembling and manufacturing the skin care device.

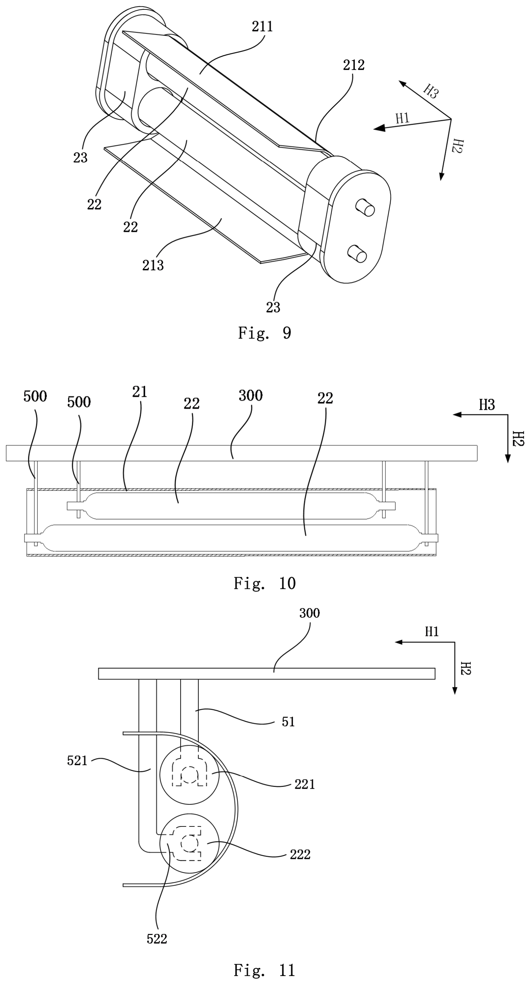

Please continue to refer to FIG. 9, which is a schematic structural view of light sources shown in FIG. 8 after installation of spacers. In some embodiments, the skin care device further includes conductive brackets 500. The conductive brackets 500 are mounted on the circuit board 300 and connected to the light sources 22, so that the light sources 22 are supported on the circuit board 300, within the reflective cavity, and electrically connected to the circuit board 300. Furthermore, the light sources 22 are lamp tubes, each light source 22 is located close to the inner wall of the reflective cavity, and the light reflector 21 is electrically connected to the circuit board 300, so as to allow the circuit board 300 to trigger or activate the lamp tubes via the light reflector 21. In this case, the skin care device further includes spacers 23. The spacers 23 are sleeved outside the at least two light sources 22 to separate the light reflector 21 from the conductive brackets 500, thereby preventing short circuits or electric leakage caused by contact between the light reflector 21 and the conductive brackets 500.

Accordingly, the skin care device further includes a conductive bracket 500. The conductive bracket 500 is mounted on the circuit board 300 and connected to the light sources 22, so that the light sources 22 are supported on the circuit board 300, within the reflective cavity, and electrically connected to the circuit board 300. Further, the light sources 22 are lamp tubes, each of the light sources 22 is located close to the inner wall of the reflective cavity, and the light reflector 21 is electrically connected to the circuit board 300, so as to allow the circuit board 300 to trigger or activate the lamp tubes via the light reflector 21. In this case, the skin care device further includes a spacer 23. The spacer 23 is sleeved outside the conductive bracket 500 to separate the light reflector 21 from the conductive bracket 500, thereby preventing short circuits or electric leakage caused by contact between the light reflector 21 and the conductive bracket 500.

In some embodiments, continuing with the example where the light sources 22 are lamp tubes. When at least one end of a lamp tube along its length direction is located outside the connection portion 212 or the light reflector 21, a spacer 23 is sleeved outside the portion of the lamp tube located outside the connection portion 212. Alternatively, the spacer 23 is sleeved outside the conductive bracket 500.

Hereinafter, examples are given in combination with the example that the conductive bracket 500 is located outside the reflective cavity, or is located to pass through the light reflector 21.

In some embodiments, one end of at least one light source 22 is located outside the reflective cavity and connected to the conductive bracket 500. The skin care device further includes a spacer 23. The spacer 23 is sleeved outside the light sources 22 and positioned between the light reflector 21 and the conductive bracket 500 to separate the light reflector 21 from the conductive bracket 500.

In some embodiments, the light reflector 21 includes a through-hole in communication with the reflective cavity, and at least one conductive bracket 500 passes through the through-hole into the reflective cavity to be connected with the light source 22. The skin care device further includes a spacer 23. The spacer 23 is sleeved outside the conductive bracket 500 and positioned within the through-hole to separate the light reflector 21 from the conductive bracket 500.

For example, continuing with the example where the light sources 22 are lamp tubes. When the conductive bracket 500 passes through the light reflector 21 into the reflective cavity to support the light source 22, a spacer 23 is sleeved outside the conductive bracket 500, and the spacer 23 is at least partially positioned within the through-hole of the light reflector 21.

In this way, the spacer 23 is configured to separate the light reflector 21 from the conductive bracket 500 so as to separate the light reflector 21 from the conductive bracket 500 so as to prevent electric leakage from the light reflector 21 to the conductive bracket 500, so as to prevent electric leakage from the light reflector 21 to the conductive bracket 500 or prevent electric leakage from the conductive bracket 500 to the light reflector 21, thereby ensuring safety during operation.

The spacers 23 being sleeved outside at least two light sources 22 is to be understood as having at least two interpretations.

The ends of multiple lamp tubes on a same side all pass through the same spacer 23. For example, among multiple lamp tubes cooperating with the same spacer 23, different lamp tubes pass through different separation holes of the spacer 23 to achieve separation. Alternatively, different lamp tubes are separated by different spacers 23. The embodiments of the present application are not limited thereto.

The spacers 23 being sleeved outside the conductive bracket 500 is to be understood as having at least two interpretations.

Adjacent conductive brackets 500 all pass through the same spacer 23. For example, among multiple conductive brackets 500 cooperating with the same spacer 23, different conductive brackets 500 pass through different separation holes of the spacer 23 to achieve electrical separation. Alternatively, different conductive brackets 500 are separated by different spacers 23. The embodiments of the present application are not limited thereto.

It can also be understood that the spacers 23 are made of insulating material. For example, the spacers 23 are made of rubber. Alternatively, the spacers are made of other insulating materials such as ceramics. The embodiments of the present application are not limited thereto.

For example, the skin care device further includes a conductive bracket 500. The conductive bracket 500 is mounted on the circuit board 300. One end of at least one light source 22 is located outside the reflective cavity and is electrically connected to the conductive bracket 500, such that the conductive bracket 500 is electrically connected to the circuit board 300.

Each end of each light source 22 in its length direction is located outside the reflective cavity, for example, outside the connection portion 212. Alternatively, the ends of only some light sources 22 in the length direction are located outside the reflective cavity, for example, outside the connection portion 212. The embodiments of the present application are not limited thereto.

Optionally, please continue to refer to FIG. 10, which is a third schematic structural view of the conductive bracket of the skin care device shown in FIG. 1. The skin care device further includes conductive brackets 500. The conductive brackets 500 are mounted on the circuit board 300 and connected to the light sources 22, to establish the electrical connection between the conductive brackets 500 and the circuit board 300. At least one of the conductive brackets 500 passes through the light reflector 21, for example, through the connection portion 212.

When at least one end of a lamp tube along its length is located inside the reflective cavity, that end of the lamp tube inside the reflective cavity is mounted via a conductive bracket 500 passing through the light reflector 21.

In some embodiments, the light sources 22 are abutted against the light reflector 21, such as the connection portion 212 of the light reflector 21, to reduce gaps between the light sources 22 and the light reflector 21, thereby making the light source assembly 200 compact, facilitating miniaturization of the skin care device.

Continuing with the example where the light sources 22 are lamp tubes, when the size of the space inside the connection portion 212 is constant, and the light sources 22 are directly abutted against the connection portion 212, the distance between the first light source 221 and the second light source 222 is increased, thereby reducing the probability of interference between the first light source 221 and the second light source 222 and improving reliability of the light sources 22. In other words, this facilitates the arrangement of the first light source 221 and the second light source 222 within the light reflector 21 without increasing, or with a slight increase in the size of the light reflector 21, which is conducive to achieving a miniaturized design.

For example, when the at least two light sources 22 include the first light source 221 and the second light source 222, and the connection portion 212 of the light reflector 21 includes a curved surface, the first light source 221 and the second light source 222 are positioned at two one-third points along the connection portion 212.

When the number of light sources 22 is more than three, multiple light sources 22 are arranged along the inner wall of the reflective cavity.

For example, when there are three light sources 22 and the connection portion 212 of the light reflector 21 includes a curved surface, the three light sources 22 are positioned at three one-fourth points along the connection portion 212. When there are four light sources 22 and the connection portion 212 of the light reflector 21 includes a curved surface, the four light sources 22 are positioned at four one-fifth points along the connection portion 212.

Continuing with the example where the light sources 22 are lamp tubes, the light reflector 21 is electrically connected to the circuit board 300 to allow the circuit board 300 to trigger, that is, activate the lamp tubes via the light reflector 21. Furthermore, it can also be understood that the light reflector 21 is reused to activate the lamp tube to eliminate the need to wind a wire (such as a filament) around a surface of the lamp tube for the activation of the lamp tube, which simplifies the structure of the skin care device and reduces the production difficulty and manufacturing cost of the skin care device.

Accordingly, in other embodiments, when the light sources 22 are positioned at a large distance from the light reflector 21, the light sources are also triggered by winding filaments around the light sources 22, which are not limited to the embodiments of the present application.

Continuing with the example where the light sources 22 are lamp tubes, all lamp tubes are of the same length, so that the brightness of light emitted from different light sources 22 is consistent, resulting in a uniform brightness at various positions of the light outlet 11.

The above provides exemplary descriptions of some optional structures for the positional arrangement of at least two light sources 22 in the embodiments of the present application. Below, the technical solutions of the embodiments of the present application are further explained and described in combination with some optional structures of the conductive brackets 500.

Please continue to refer to FIG. 11, which is a schematic structural view of the conductive brackets of the skin care device shown in FIG. 2. Continuing with the example where the at least two light sources 22 include the first light source 221 and the second light source 222. The circuit board 300, the first light source 221, and the second light source 222 are sequentially arranged along the second direction H2.

In this example, the skin care device further includes a first conductive bracket 51 and a second conductive bracket 52. The first conductive bracket 51 and the second conductive bracket 52 correspond to the conductive brackets 500 described above. The first conductive bracket 51 is connected between the first light source 221 and the circuit board 300. The second conductive bracket 52 includes a first portion 521 and a second portion 522 arranged to be connected at an angle. The first portion 521 is connected to the circuit board 300. The first portion 521 is arranged at an interval with the first conductive bracket 51 along the first direction H1 and/or the third direction H3. The first direction, the second direction, and the third direction are mutually orthogonal. The second portion 522 is arranged at an interval with the first conductive bracket 51 along the second direction H2. The second portion 522 is connected to the second light source 222.

Such an arrangement allows at least two light sources 22 of a same length to be individually mounted on the same circuit board 300, and supported within the reflective cavity to achieve flexible control of at least two light sources 22, so as to increase the light output power (e.g., power for hair removal) and/or the diversity of light output functions.

For example, the circuit board 300, the first light source 221, and the second light source 222 are arranged from top to bottom along the thickness direction of the skin care device, the first conductive bracket 51 extends downward from top to bottom. The first portion 521 of the second conductive bracket 52 also extends downward from top to bottom, and the first portion 521 is arranged at an interval with the first conductive bracket 51 in the front-back or left-right direction.

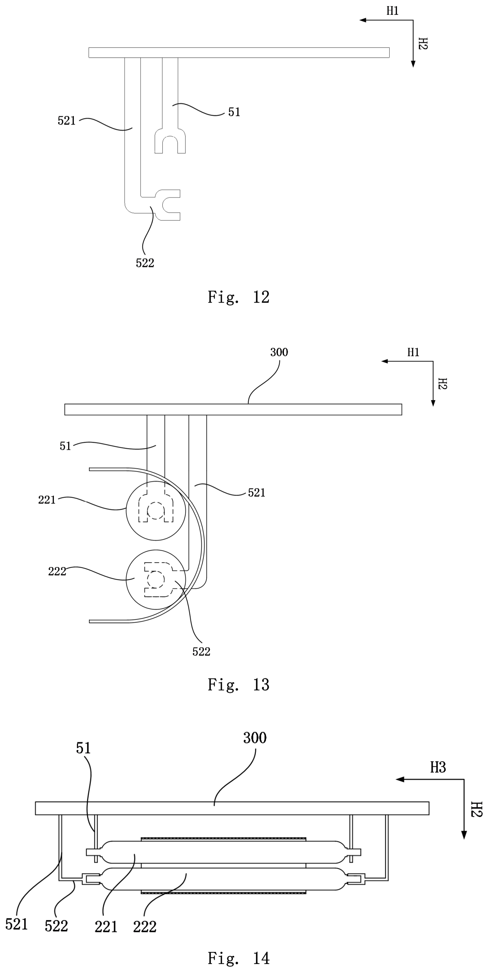

For example, please refer to FIGS. 11 to 14 as a whole, where FIG. 12 is a schematic structural view of the conductive brackets shown in FIG. 11, FIG. 13 is a fourth schematic structural view of the conductive brackets of the skin care device shown in FIG. 1, and FIG. 14 is a fifth schematic structural view of the conductive brackets of the skin care device shown in FIG. 1. The first portion 521 is located on a side of the first conductive bracket 51 close to or away from the light outlet 11. Alternatively, the first portion 521 is located on a side of the first conductive bracket 51 along the third direction H3, which are not limited to the embodiments of the present application.

In some embodiments, at least one of a width of the first conductive bracket 51 and a width of the first portion 521 is equal to a width of the second portion 522. Therefore, resistances of the respective conductive bracket 500 and/or at various positions of each conductive bracket 500 are substantially equal, so that current transmission between the circuit board 300 and each light source 22 is uniform, resulting in a uniform brightness at various positions of the light outlet 11.

It can be that only the width of the first conductive bracket 51 is equal to the width of the second portion 522. Alternatively, the width of the first portion 521 is equal to the width of the second portion 522. Alternatively, the width of the first conductive bracket 51, the width of the first portion 521 are both equal to the width of the second portion 522, which are not limited to the embodiments of the present application.

In some embodiments, the light sources 22 are lamp tubes arranged to extend along the third direction H3. The first direction, the second direction, and the third direction are mutually orthogonal. All lamp tubes are of the same length in the third direction H3, so that the brightness of light emitted by different light sources 22 is consistent, resulting in a uniform brightness at various positions of the light outlet 11.

In some embodiments, each light source 22 is arranged close to the inner wall of the reflective cavity, and a distance between each light source 22 and the inner wall of the reflective cavity is less than or equal to 0.3 mm.

For example, the distance between each light source 22 and the inner wall of the reflective cavity is 0.3 mm, 0.287 mm, 0.266 mm, 0.214 mm, 0.201 mm, 0.177 mm, 0.15 mm, 0.123 mm, 0.1 mm, 0.081 mm, 0.05 mm, or 0 mm. When the distance between each light source 22 and the inner wall of the reflective cavity is 0 mm, it can be understood that the light source 22 is abutted against the inner wall of the reflective cavity.

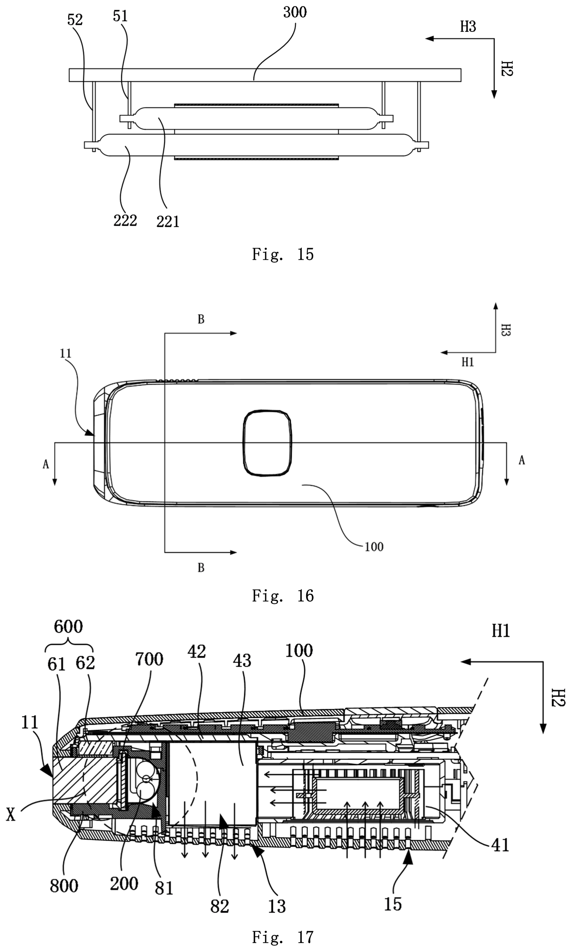

Please continue to refer to FIG. 15, which is a sixth schematic structural view of the conductive brackets of the skin care device shown in FIG. 1. In some other embodiments, the light sources 22 are of different lengths.

For example, continuing with the example where at least two light sources 22 include the first light source 221 and the second light source 222, and the circuit board 300, the first light source 221, and the second light source 222 are sequentially arranged along the second direction H2. The first light source 221 and the second light source 222 are lamp tubes arranged to extend along the third direction H3. The first direction, the second direction, and the third direction are mutually orthogonal. The second light source 222 protrudes beyond the first light source 221 at each end along the third direction H3. The skin care device further includes a first conductive bracket 51 and a second conductive bracket 52. The first conductive bracket 51 is connected between the first light source 221 and the circuit board 300. The second conductive bracket 52 is connected between the second light source 222 and the circuit board 300. The second conductive bracket 52 is arranged at an interval with the first conductive bracket 51 along the third direction H3. Accordingly, it can also be understood that the second conductive bracket 52 is located on a side of the adjacent first conductive bracket 51 away from the first light source 221, or, in other words, on an outer side of the adjacent first conductive bracket 51.

The above provides illustrative examples in which all the conductive brackets 500 are connected to one circuit board 300 located on the same side of the light source assembly 200. Hereinafter, the technical solutions of the embodiments of the present application are further described with reference to examples in which the conductive brackets 500 are connected to the circuit boards 300 located on different sides of the light source assembly 200.

The light source 22 includes a first light source 221 and a second light source 222 arranged along the second direction H2. The circuit board 300 includes a first sub-circuit board 31 and a second sub-circuit board 32. The first sub-circuit board 31 is located on a side of the first light source 221 away from the second light source 222, and the second sub-circuit board 32 is located on a side of the second light source 222 away from the first light source 221. The skin care device further includes a first conductive bracket 51 and a second conductive bracket 52. The first conductive bracket 51 is connected between the first sub-circuit board 31 and the first light source 221, and the second conductive bracket 52 is connected between the second sub-circuit board 32 and the second light source 222.

It can be understood that the above designs allow at least two light sources 22 of a same length individually supported within the reflective cavity to achieve flexible control of at least two light sources 22, so as to increase the light output power (e.g., power for hair removal) and/or the diversity of light output functions.

The above provides illustrative examples of the light source assembly 200 in the embodiments of the present application. Hereinafter, further illustrative examples are provided in combination with some other optional structures in the embodiments of the present application.

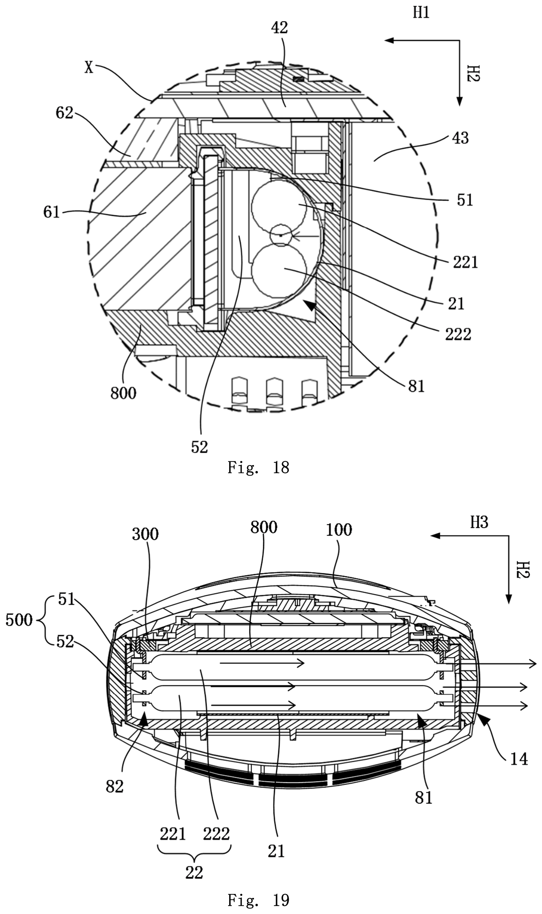

Please continue to refer to FIGS. 16 to 18, where FIG. 16 is a top view of the skin care device shown in FIG. 1, FIG. 17 is a cross-sectional view of the skin care device shown in FIG. 16 taken along line A-A, and FIG. 18 is an enlarged view of part X in FIG. 17. The skin care device further includes a cooling assembly 600 mounted on the housing 100 for cooling the skin.

For example, the cooling assembly 600 includes a light guide 61 and a thermoelectric cooling plate 62. The light guide 61 is arranged at the light outlet 11 to transmit light emitted by the light sources 22. The thermoelectric cooling plate 62 is thermally connected to the light guide 61. For example, the thermoelectric cooling plate 62 is in direct contact with the light guide 61 or contacts the light guide 61 via a thermally conductive medium such as a silicone-based thermal grease.

In this way, the thermoelectric cooling plate 62 cools the light guide 61, and the cooled light guide 61 is exposed at the light outlet 11 and in contact with the skin to allow the light guide 61 not only to transmit the light from the light sources 22 for skin care but also to provide a cooling effect to the user via the thermoelectric cooling plate 62.

It can also be understood that the light source assembly 200 serves as a main heat source of the skin care device, the light guide 61 is located on a light output side of the light source assembly 200, and the light guide 61 is cooled through the thermoelectric cooling plate 62, so that part of the heat from the light source assembly 200 is dissipated, preventing damage to the light source assembly 200 due to high temperature.

The thermoelectric cooling plate 62, also called a semiconductor thermoelectric cooling plate, utilizes the Peltier effect of semiconductor materials. When a direct current passes through a thermocouple formed by two different semiconductor materials connected in series, heat is absorbed at one end of the thermocouple and released at the other end for the purpose of cooling.

The light guide 61 is made of sapphire or other light-transmitting materials, which are not limited to the embodiments of the present application.

The skin care device further includes an optical filter 700. The optical filter 700 is arranged between the light source assembly 200 and the light guide 61 and is configured to filter light emitted from the light source assembly 200 towards the light guide 61.

For example, the optical filter 700 is configured to transmit light with wavelengths between 420 nm and 1200 nm, thereby forming IPL (intense pulsed light) to irradiate skin of the user. In actual use, the wavelength of light required for hair removal is in a range of 420 nm and 1200 nm, and the wavelength of light required for skin rejuvenation is in a range of 600 nm and 1200 nm. Thus, the skin care device provides cooling, skin rejuvenation, and hair removal functions.

Hereinafter, the technical solutions of the embodiments of the present application are further illustrated with reference to some optional structures of the heat dissipation assembly 400 in the embodiments of the present application.

A heat dissipation channel 12 is arranged inside the housing 100. The heat dissipation assembly 400 includes a fan 41. The fan 41 is configured to drive air in the heat dissipation channel 12 to flow in a preset direction, so as to dissipate heat from the light source assembly 200 and/or the cooling assembly 600.

That is, the fan 41 is configured to dissipate heat only from the light source assembly 200, to dissipate heat only from the cooling assembly 600, or to dissipate heat from both the light source assembly 200 and the cooling assembly 600, which are not limited to the embodiments of the present application.

For example, when the heat dissipation assembly 400 is configured to dissipate heat from the cooling assembly 600, the heat dissipation assembly 400 further includes a heat-conducting member 42 and a heat sink 43. The heat-conducting member 42 includes a first end and a second end. The first end is thermally connected to the thermoelectric cooling plate 62. For example, the first end is in direct contact with the thermoelectric cooling plate 62 or contact with the thermoelectric cooling plate 62 via a thermally conductive medium, such as silicone-based thermal grease. The heat sink 43 is thermally connected to the second end. For example, the first end is in direct contact with the thermoelectric cooling plate 62 or contact with the thermoelectric cooling plate 62 via a thermally conductive medium, such as silicone-based thermal grease. The heat sink 43 is located on a side of the light reflector 21 away from the light outlet 11 and at least partially within the heat dissipation channel 12.

In this way, the heat-conducting member 42 transfers heat from the thermoelectric cooling plate 62 to the heat sink 43, and the fan 41 rapidly air-cools the heat sink 43 by driving air in the heat dissipation channel 12 to flow.

Further, when the heat dissipation assembly 400 is configured to dissipate heat from both the light source assembly 200 and the cooling assembly 600, the heat dissipation channel 12 includes a first heat dissipation channel and a second heat dissipation channel.

The first heat dissipation channel is connected between an air port of the housing 100, such as a first air port 13, and the fan 41. At least one of the light sources 22 and the light reflector 21 is located within the first heat dissipation channel, so that the fan 41 dissipates heat from the light sources 22 and/or the light reflector 21 within the first heat dissipation channel.

The second heat dissipation channel is connected between an air port of the housing 100, such as a second air port 14, and the fan 41. The heat sink 43 is located in the second heat dissipation channel. The heat-conducting member 42 transfers heat from the thermoelectric cooling plate 62 to the heat sink, and the second heat dissipation channel is air-cooled by the fan 41 for the purpose of heat dissipation.

In some embodiments, the second heat dissipation channel is independent of the first heat dissipation channel to achieve zoned cooling within the skin care device, thereby ensuring an effective heat dissipation for both the light source assembly 200 and the cooling assembly 600.

In some embodiments, the second heat dissipation channel and the first heat dissipation channel are further partially interconnected, which are not limited to the embodiments of the present application.

By way of example, please continue to refer to FIG. 19, which is a cross-sectional view of the skin care device shown in FIG. 16 taken along line B-B. The skin care device further includes a mounting bracket 800. The mounting bracket 800 is arranged within the housing 100. The mounting bracket 800 includes a first mounting cavity 81, a connection channel 82, and a second mounting cavity 83.

The light sources 22 and the light reflector 21 are located within the first mounting cavity 81. One end of the connection channel 82 is in communication with the first mounting cavity 81, and the other end of the connection channel 82 is connected to an air outlet of the fan 41, so that the first mounting cavity 81 and the connection channel 82 form at least a portion of the first heat dissipation channel.

The heat sink 43 is at least partially arranged within the second mounting cavity 83. The second mounting cavity 83 is in communication with the air outlet of the fan 41 to form at least a portion of the second heat dissipation channel.

The mounting bracket 800 includes at least two brackets. For example, the mounting bracket 800 includes a first bracket and a second bracket. The first bracket and the second bracket are spliced together to form the first mounting cavity 81, the connection channel 82, and the second mounting cavity 83 stated above, so as to reduce the manufacturing difficulty of the mounting bracket 800.

Corresponding slots, ribs, or the like are arranged inside the mounting bracket 800 to facilitate the clamping of the light reflector 21, the optical filter 700, and other components, which are not limited to the embodiments of the present application.

The mounting bracket 800 further includes corresponding openings to allow the light guide 61, the thermoelectric cooling plate 62, and other components to pass through the mounting bracket 800, which are not limited to the embodiments of the present application.

The fan 41 is a centrifugal fan, an axial fan, or a cross-flow fan, which are not limited to the embodiments of the present application.

For example, when the fan 41 is a centrifugal fan, an air inlet of a volute of the centrifugal fan is in communication with an air port of the housing 100, such as a third air port 15. An inlet of the connection channel 82 and an inlet of the second mounting cavity 83 are arranged in parallel at the air outlet of the volute of the centrifugal fan, so that the centrifugal fan drives the air outside the housing 100 to enter the volute of the centrifugal fan. The air is then divided into two paths from the volute of the centrifugal fan, that is, one path passing through the connection channel 82 and the first mounting cavity 81 to be discharged for air cooling of the light sources 22 and/or the light reflector 21, and the other path passing through the second mounting cavity 83 for air cooling of the heat sink 43 or the cooling assembly 600.

In some embodiments, the heat sink 43 includes multiple fins. The fins are protruded from a side of the second end along the second direction H2 and are arranged within the heat dissipation channel 12. The fan 41 is located on a side of the fins away from the light reflector 21 along the first direction H1. A height of the fins along the second direction H2 is greater than or equal to a height of the light source assembly 200 along the same direction.

It can be understood that, compared with the arrangement where the fan 41 and the fins are arranged along the second direction H2 behind the light source assembly 200, in the embodiments of the present application, it can be understood that the fan 41 is moved in a direction away from the light source assembly 200, which allows the fins to be made larger along the second direction H2, thereby increasing a contact area between the heat sink 43 and the air and greatly enhancing the heat dissipation performance of the heat sink 43.

## Claims

1. A skin care device, wherein the skin care device comprises:
a housing, a light outlet being formed on the housing;
a light source assembly arranged inside the housing, the light source assembly comprising a light reflector and at least two light sources, the light outlet being located on a side of the light sources along a first direction, the light reflector being at least partially located on a side of the light sources facing away from the light outlet to reflect part of light emitted from the light sources towards the light outlet;
a circuit board arranged within the housing, the circuit board being arranged on at least one side of the light sources along a second direction, the second direction being perpendicular to the first direction, the circuit board being electrically connected to the light sources; and
a heat dissipation assembly arranged inside the housing, the heat dissipation assembly being at least partially located on a side of the light reflector facing away from the light outlet.

2. The skin care device according to claim 1, wherein the skin care device further comprises a conductive bracket, the conductive bracket is mounted on the circuit board and connected to the light sources, such that the light sources are supported on the respective circuit board and electrically connected to the circuit board.

3. The skin care device according to claim 2, wherein,
a plurality of conductive brackets are provided, ends of all the conductive brackets connected to the circuit board are located on a same side of the light source assembly along the second direction; or
a plurality of conductive brackets are provided, ends of all the conductive brackets connected to the circuit boards are located on different sides of the light source assembly along the second direction.

4. The skin care device according to claim 2, wherein the light sources comprise a first light source and a second light source, and the conductive brackets comprise a first conductive bracket, a second conductive bracket, and a third conductive bracket, and wherein,
a positive electrode of the first light source and a positive electrode of the second light source are both electrically connected to the third conductive bracket, a negative electrode of the first light source is electrically connected to the first conductive bracket, and a negative electrode of the second light source is electrically connected to the second conductive bracket; or
the negative electrode of the first light source and the negative electrode of the second light source are both electrically connected to the third conductive bracket, the positive electrode of the first light source is electrically connected to the first conductive bracket, and the positive electrode of the second light source is electrically connected to the second conductive bracket.

5. The skin care device according to claim 2, wherein different light sources are mounted on the circuit board through different conductive brackets.

6. The skin care device according to claim 5, wherein a distance between two adjacent conductive brackets configured to support different light sources is greater than or equal to 0.8 mm and less than or equal to 10 mm.

7. The skin care device according to claim 1, wherein the plurality of light sources are parallel to each other; and/or distances between the plurality of light sources and the light outlet are equal.

8. The skin care device according to claim 1, wherein a reflective cavity is provided at the light reflector, an opening of the reflective cavity faces the light outlet, the at least two light sources are mounted within the reflective cavity, and the at least two light sources are disposed at an interval along the second direction.

9. The skin care device according to claim 8, wherein along the second direction, the reflective cavity is symmetrical about a first axis, the first axis is parallel to the first direction, and all the light sources are symmetrically arranged about the first axis; and/or
in a case that a number of the light sources is greater than three, all the light sources are arranged along an inner wall of the reflective cavity in the second direction.

10. The skin care device according to claim 8, wherein the light reflector comprises a first straight portion, a connection portion, and a second straight portion sequentially connected, the first straight portion and the second straight portion are arranged at an interval along the second direction, and the connection portion faces the light outlet such that the first straight portion, the connection portion, and the second straight portion are enclosed to form the reflective cavity.

11. The skin care device according to claim 10, wherein,
the connection portion comprises a curved surface, one end of the curved surface in a circumferential direction is connected to the first straight portion and another end of the curved surface in the circumferential direction is connected to the second straight portion; or
the connection portion comprises at least two light-concentrating curved surfaces, each of the light-concentrating curved surfaces is arranged corresponding to a corresponding one of the light sources.

12. The skin care device according to claim 10, wherein the connection portion is arranged to surround the at least two light sources, and one end of the connection portion connected to the first straight portion and one end of the connection portion connected to the second straight portion are both protruded from a side of the at least two light sources close to the light outlet.

13. The skin care device according to claim 12, wherein the light sources are lamp tubes, a length direction of the lamp tubes, the first direction, and the second direction are perpendicular to each other, at least one end of each of the lamp tubes along the length direction is located outside the connection portion; and
wherein along the length direction of the lamp tubes, a length of a portion at each end of each of the lamp tubes located outside the connection portion is less than or equal to one-fifth of a length of each of the lamp tubes.

14. The skin care device according to claim 8, wherein the light sources are each abutted against the light reflector.

15. The skin care device according to claim 8, wherein the light sources are lamp tubes, and the light reflector is electrically connected to the circuit board, allowing the circuit board to trigger activating the lamp tubes via the light reflector.

16. The skin care device according to claim 8, wherein the skin care device further comprises a conductive bracket, the conductive bracket is mounted on the circuit board, one end of at least one of the light sources is located outside the reflective cavity and is connected to the conductive bracket, so that the light sources are connected to the circuit board through the conductive bracket; or
the skin care device further comprises conductive brackets, the conductive brackets are mounted on the circuit board and connected to the light sources, so that the light sources are connected to the circuit board through the conductive brackets; wherein at least one of the conductive brackets passes through the light reflector.

17. The skin care device according to claim 8, wherein the at least two light sources comprise a first light source and a second light source, the circuit board, the first light source, and the second light source are sequentially arranged along the second direction, the skin care device further comprises:
a first conductive bracket connected between the first light source and the circuit board; and
a second conductive bracket comprising a first portion and a second portion arranged to be connected at an angle, wherein the first portion is connected to the circuit board, the first portion is arranged at an interval with the first conductive bracket along the first direction and/or the third direction, the first direction, the second direction, and the third direction are mutually orthogonal, the second portion is arranged at an interval with the first conductive bracket along the second direction, and the second portion is connected to the second light source.

18. The skin care device according to claim 17, wherein, the first portion is located on a side of the first conductive bracket close to or away from the light outlet; and/or
at least one of a width of the first conductive bracket and a width of the first portion is equal to a width of the second portion; and/or
the light sources are lamp tubes arranged to extend along the third direction, the first direction, the second direction, and the third direction are mutually orthogonal, and all the lamp tubes are of a same length along the third direction; and/or
the light sources are each arranged close to an inner wall of the reflective cavity, and a distance between each of the light sources and the inner wall of the reflective cavity is less than or equal to 0.3 mm.

19. The skin care device according to claim 8, wherein the at least two light sources comprise a first light source and a second light source, the circuit board, the first light source, and the second light source are sequentially arranged along the second direction, the first light source and the second light source are lamp tubes arranged to extend along a third direction, the first direction, the second direction, and the third direction are mutually orthogonal, each end of the second light source along the third direction protrudes beyond the first light source; the skin care device further comprises:
a first conductive bracket connected between the first light source and the circuit board; and
a second conductive bracket connected between the second light source and the circuit board, the second conductive bracket is arranged at an interval with the first conductive bracket along the third direction.

20. The skin care device according to claim 8, wherein,
the light sources comprise a first light source and a second light source arranged along the second direction;
the circuit board comprises a first sub-circuit board and a second sub-circuit board, the first sub-circuit board is located on a side of the first light source facing away from the second light source, the second sub-circuit board is located on a side of the second light source facing away from the first light source; and
the skin care device further comprises a first conductive bracket and a second conductive bracket, the first conductive bracket is connected between the first sub-circuit board and the first light source, the second conductive bracket is connected between the second sub-circuit board and the second light source.

21. The skin care device according to claim 8, wherein the skin care device further comprises a conductive bracket, the conductive bracket is mounted on the circuit board and connected to the light sources so that the light sources are supported on the circuit board, located within the reflective cavity, and electrically connected to the circuit board;
the light sources are lamp tubes, and the light sources are each arranged close to an inner wall of the reflective cavity, the light reflector is electrically connected to the circuit board so that the circuit board is capable of activating the lamp tubes through the light reflector; and
the skin care device further comprises a spacer, the spacer being sleeved outside the light sources or sleeved outside the conductive brackets, and the spacer being configured to separate the light reflector from the conductive brackets.

22. The skin care device according to claim 8, wherein the skin care device further comprises a conductive bracket and a spacer, the conductive bracket is mounted on the circuit board, one end of at least one of the light sources is located outside the reflective cavity and connected to the conductive bracket, the spacer is sleeved outside the light sources and located between the light reflector and the conductive bracket; or
the skin care device further comprises conductive brackets and a spacer, the conductive brackets are mounted on the circuit board, the light reflector includes a through-hole in communication with the reflective cavity, at least one of the conductive brackets passes through the through-hole into the reflective cavity to connect with the light sources, the spacer is sleeved outside the conductive brackets and located within the through-hole.

23. The skin care device according to claim 1, wherein the at least two light sources comprise a first light source and a second light source, a distance between the first light source and the light outlet is greater than a distance between the second light source and the light outlet, and a power of the first light source is greater than a power of the second light source.

24. The skin care device according to any one of claims 1 to 23, wherein the skin care device further comprises a cooling assembly mounted on the housing to provide a cooling effect to the skin.

25. The skin care device according to claim 24, wherein heat dissipation channels are formed in the housing, and the heat dissipation assembly comprises a fan configured to drive air in the heat dissipation channels to flow in a preset direction to dissipate heat from the light source assembly and/or the cooling assembly.

26. The skin care device according to claim 25, wherein the cooling assembly comprises a light guide and a thermoelectric cooling plate, the light guide is arranged at the light outlet to transmit light emitted from the light sources, the thermoelectric cooling plate is thermally connected to the light guide; wherein the heat dissipation assembly further comprises:
a heat-conducting member comprising a first end and a second end, the first end is thermally connected to the thermoelectric cooling plate; and
a heat sink thermally connected to the second end, the heat sink is located on the side of the light reflector facing away from the light outlet and at least partially within the heat dissipation channels.

27. The skin care device according to claim 26, wherein the heat dissipation channels comprise:
a first heat dissipation channel connected between an air outlet of the housing and the fan, at least one of the light sources and light reflector is arranged in the first heat dissipation channel; and
a second heat dissipation channel connected between the air outlet of the housing and the fan, and the heat sink is arranged within the second heat dissipation channel.

28. The skin care device according to claim 27, the skin care device further comprises a mounting bracket arranged within the housing, and a first mounting cavity, a connection channel, and a second mounting cavity are formed in the mounting bracket; wherein,
the light sources and the light reflector are arranged within the first mounting cavity, one end of the connection channel is in communication with the first mounting cavity and another end of the connection channel is in communication with an air outlet of the fan so that the first mounting cavity and the connection channel form at least part of the first heat dissipation channel; and
the heat sink is at least partially arranged within the second mounting cavity, and the second mounting cavity is in communication with the air outlet of the fan to form at least part of the second heat dissipation channel.

29. The skin care device according to claim 26, wherein the heat sink comprises a plurality of fins protruding from a side of the second end along the second direction and positioned within the heat dissipation channels; and
wherein the fan is located on a side of the fins facing away from the light reflector along the first direction, and a height of the fins along the second direction is greater than or equal to a height of the light source assembly along the second direction.

30. The skin care device according to any one of claims 1 to 23, wherein the skin care device is a hair removal device or a skin rejuvenation device.
